# EUROPEAN PATENT SPECIFICATION

(11) **EP 2 950 830 B1**
(45) Date of publication and mention of the grant of the patent: **28.12.2016**
(21) Application number: 14702015.0
(22) Date of filing: 30.01.2014
(51) Int. Cl.: A61L 24/00, C08G 83/00, C09J 201/00

(54) **POLYMER ADHESIVE**
POLYMERKLEBSTOFF
ADHÉSIF POLYMÈRE

(30) Priority: 31.01.2013 EP 13153503
(43) Date of publication of application: 09.12.2015
(73) Proprietor: University College Dublin, National University of Ireland, Dublin, Dublin 4 (IE)
(72) Inventor: WANG, Wenxin, Galway (IE); ZHENG, Yu, Galway (IE); ZHANG, Hong, Galway (IE); PANDIT, Abhay, Galway (IE); DaCOSTA, Mark, Galway (IE); BRE, Ligia Pereira, Galway (IE)
(74) Representative: Tomkins & Co
(86) International application number: PCT/EP2014/051779
(87) International publication number: WO 2014/118266

(56) References cited:
- WO-A2-2007/087256
- US-A1- 2010 113 828
- US-A1- 2011 082 498

## Description

### Field of the Invention

The invention relates to the provision of biocompatible and biodegradable tissue adhesives which are based on dendritic/hyperbranched polymers. The tissue adhesives of the invention may be used as a wet tissue adhesive, for example, as a sternum bone closure adhesive, an incisional or laceration wound closure adhesive.

### Background to the Invention

Wound closure and healing are essential for achieving optimal outcomes for all surgical patients. The healing process is facilitated by proper closure of the surgical wound. Traditionally, wound healing has been a particularly invasive practice. Devices such as sutures and staples have been used for many years. These methods can be traumatic to the patient. The ideal incision closure should be simple, effective, safe, rapid, inexpensive, painless, cosmetic and bactericidal. Although there has been great research into mechanical methods there is still the possibility of localised infection and inflammation of the wound area. Infection can arise from the presence of an external body physically carrying antibiotic - resistant bacteria detrimental to the patient's health. In general, the use of tissue adhesives can significantly decrease health-care costs and is preferred by patients. Adhesives provide a needle-free method of wound closure, an important consideration in light of the risks presented by blood-borne viruses. In addition, adhesives do not require local anaesthetics.

With a fast curing adhesive, the wound can be closed quickly and it wears off after four or five days, which makes the process simpler and better. The topical skin adhesive adheres to the skin and itself, forming a clean strong adherent bond that holds the edges of skin wounds together so that the wounds can heal normally underneath the film. When intact, the film acts as a microbial barrier to protect the wound from potential contamination. Topical tissue adhesives slough from the skin in five to ten days as the skin heals underneath and it is no longer required, thus eliminating the need for non-absorbable suture or staple removal. Current adhesives available in the market for wound healing can be categorized into three groups: fibrin glue, synthetic polymer adhesive and biomimetic polymer adhesive. Fibrin sealants or "fibrin glues," which are the end product of the coagulation cascade, serve as a primary hemostatic plug as well as a matrix to enhance wound healing. In contrast to synthetic adhesives, fibrin sealants have the advantage of being biocompatible and biodegradable. The fibrin clot is resorbed within days to weeks as a part of the normal wound-healing process. As such, they are not associated with inflammation, foreign body reactions, tissue necrosis, or extensive fibrosis. However, fibrin glues widely used in surgical practice do not provide significant adhesive strength, optimal application requires dry substrates, and is high cost and associated with risk of viral transmission.

Two mainly synthetic polymer adhesive available are cyanoacrylates and polyethylene glycol based products. Topical cyanoacrylate skin adhesives offer many advantages over traditional wound closure devices. The cyanoacrylates function as waterproof occlusive dressings, have microbial barrier properties against gram-positive organisms, and may also reduce infections. Topical skin adhesives, therefore, provide not only primary closure but an effective microbial barrier throughout this critical time period. Cyanoacrylates are synthetic glues that rapidly polymerize on contact with water or blood. n-Butyl-cyanoacrylates have shorter molecular chain lengths, this allows the product to require only one layer and dry significantly faster while still maintaining its efficacy and strength. These topical skin adhesives dry in 30 seconds, allow for a one-layer application, and have a low exothermic reaction. 2-octyl cyanoacrylate (Dermabond®) have longer molecular chain lengths, this allows the product to have more flexibility and smooth appearance during healing while still maintaining its efficacy and strength. They do however take a longer time to dry (polymerize) as a result of the two layer application and longer molecular chain lengths. 2-octyl cyanoacrylate degrades much more slowly due to its longer organic backbone which slows the degradation of the adhesive enough to remain below the threshold of tissue toxicity. Due to the toxicity issues of cyanoacrylate, the use of 2-octyl cyanoacrylate for sutures is preferred. Cyanoacrylate adhesives appear to have greater performance in external tissue bonding applications, particularly in medical emergency, but it is moisture sensitive and toxic to the exposed wound site.

Polyethylene glycol (PEG) polymers are hydrogels that are used for tissue adhesion. FocalSeal-L is a water-soluble, bioabsorbable compound that requires a significant amount of time for photoactivation, which is a limitation for its use in hemostasis. Another PEG polymer, CoSeal is the first PEG-based sealant approved by FDA for use in the US, does not require the same activation source and is currently being used in Europe for similar purposes. FocalSeal has thus far proved useful in decreasing air leaks after major thoracic surgery but has not yet been used in the GI endoscopic domain. Coseal allows surgeons to operate vascular-related surgeries with improved safety and efficiency. However, the polyethylene glycol (PEG) glues have the disadvantages of being applied as a two-step process and some still require UV photo-crosslinking. Recently, lots of researches have been done work on this part, for instance, acrylic adhesives, hydrocolloid adhesives, hydrogel adhesives, polyurethane adhesives and soft silicone adhesives, but most of them have to be used on wound dressings and regrettably, none of them is a perfect tissue adhesive for internal wound repair.

Biomimetic adhesives are seen as an alternative to biological adhesives. Biomimetic adhesives are designed to mimic the crosslinking process that occurs naturally in blood clotting. There has been increased interest and research into biomimetic adhesives as they have shown the appealing adhesive properties in wet environments. However, there are currently no biomimetic skin adhesive products on the market today.

A median sternotomy the most commonly performed procedure in cardiothoracic surgery with over 1.4 million performed annually worldwide. This procedure is performed by separating the tissue over the sternum with a scalpel and then dividing the sternum with an electric saw. During the operation the sternum is held open with a sternal retractor. After the procedure the sternum must be held together to allow for healing. The quality with which the sternum is held together is of vital important to the healing process as it affects pain, the possibility of morbidity and mortality, healing time and the strength of the bone when repaired. The standard methods for closing this wound use stainless steel wire and pins which pierce the bone. However there are a number of associated problems. Aside from the discomfort endured by patients who use stainless steel wire, there is an associated morbidity of 0.5-8% and mortality rate 10-40% with this technique. These methods are not biodegradable and require a second surgery for removal which increases the likelihood of surgical complications, they are traumatic and cause damage to the bone and can delay emergency surgery in the case of complications which is most likely to be necessary in the first 24 hours after the surgery. The first method of closure is to pierce the bone, and suture the bone closed using stainless steel wire. This is normally done using 6 wires. This is the standard of care despite evidence of pathologic sternal displacement (>2 mm) during physiologic distracting forces (coughing). Unstable median sternotomy closure can lead to significant post-operative morbidity. Functional recovery, respiration, pain, sternal dehiscence and infection are all influenced by post-operative sternal stability. The use of this method severely reduces patient's mobility and strength for eight to twelve weeks following the surgery. After this period patients still need to be extremely careful. A more efficient method to replace this invasive and impractical method of closure has long been sought by doctors. Research recently published by Global Analysts Inc placed the future market potential for wound closure technologies at $2.08 billion by 2015. The same research also showed that skin adhesives and sealants have experienced a 10-20% growth rate in the past two years. Also the bone cement and accessory market is directly related to the number of hip, knee, shoulder, elbow, digit, ankle, and wrist arthroplasty procedure performed annually. Due to better living conditions and improvements in medical care the average global age is always increasing. Because of this it is predicted that the number of patients requiring bone cement treatments will increase driving up the bone cement market. The US market for orthopaedic biomaterials, of which bone cements are a part, is expected to grow by 12% per annum over the next four years.

Adhesives, especially bone adhesives, are resorbed and degraded to non-toxic products after fulfilling their function in contact with the living organism. The use of such bone adhesives has found growing interest in all fields of medicine in the last 50 years. The first bone adhesive was described in 1931 by Hedri and was a mixture of collagen and fibrous protein 'ossocol'. Good initial bonding strength and healing of fractures were reported, but severe allergic reactions prevented its use. Afterwards, a lot of researches were carried out on epoxy resins, cyanoacrylates, polyurethanes, polymethylmethacrylates (PMMA), fibrin adhesives, gelatin-resorcin-aldehydes, protein-aldehyde systems, peptide-based adhesives, adhesives based on oligo- and polylactone and alkylenebis(oligolactoyl)-methacrylates. In spite of the large amount of work which has been done in the last decade on tissue and bone adhesives, it is evident that the development of a successful bone adhesive is in its very early stages. Nowadays, widely used biodegradable materials as bone adhesives include PGA, PLLA, PDLLA, PGA/TMC, PDS and PBHBA. Research studies reveal that they are safe for majority of ankle fractures, but not suitable for comminuted and unstable fractures and most bone adhesives being used now are cements with minimal adhesive properties, which bond surfaces together by interlocking into pores and imperfections in the surfaces of the underlying bone. Canadian researchers have pioneered a new bone cement, called Kryptonite®, a biocompatible polymer, which is widely used in healing bone damage. This bone cement has also been used to aid repair in a number of median sternotomy cases. It has been shown to provide an improved sternal stability over using wires alone, which will improve healing time and comfort. It also allows for increased mobility shortly following surgery. KRYPTONITE™ which allows the breast bone to adhere together within hours instead of several weeks as is the case when using traditional methods, is the first adhesive which have found a way to help open heart surgery patients to recover better and faster. It is suggested that the use of adhesive in chest surgery increased mechanical strength of the breastbone closure by 5 to 10 times that of wires alone, reduces the normal recovery time by half and patients have less physical disability in the first six weeks after surgery and can breathe deeply sooner. Patients are able to cough with less discomfort and require significantly less medication such as narcotics to manage pain. However the bonding strength in the first 24 hours is too high to allow for easy emergency surgery. A median sternotomy is a procedure which makes a vertical inline incision in the sternum allowing surgeons access the heart and lungs. It is the most commonly performed procedure in cardiothoracic surgery with over 750,000 cases performed per year in the US. Following the surgery the chest is usually closed using stainless steel wire and pins placed through the sternum. A more efficient method to replace this invasive and impractical method of closure has long been sought by doctors as there is an associated morbidity (5%) and a mortality rate if failure or dehiscence occurs (25%). Also post operative complications often occur shortly after the initial procedure requiring the chest to be reopened in which case the steel wire or pins could cause fatal delay. As well as of this a non biodegradable method of sternum bone closure will require a second surgery which will increase the likelihood of fatal harm. An alternative to this method that can reduce discomfort, healing time and allow for easy access within 24 hours is needed. The aim of this project is to develop a biodegradable bone adhesive tailored for this application. Recently, more research has been carried out to develop adhesives for effective bone repair. Biodegradable bone adhesives are materials that induce complete bone regeneration and disappear as soon as the bone recovers, since they are gradually self-degrading in the body. Biodegradable adhesives thus serve as an alternative to conventional adhesives and have the advantage of not being necessary to remove once the fracture has healed. A number of biodegradable materials have been shown to be safe as bone adhesives for the majority of ankle fractures but unsafe for unstable or comminuted fractures. These biomaterials include polyglycolic acid (PGA), poly-L-lactic acid (PLLA), poly-DL-lactic acid (PDLLA), PGA/trimethylenecarbonate copolymers (PGA/TMC), poly-p-dioxanone (PDS) and poly-beta-hydroxybutyric acid (PBHBA). However, most bone adhesives being used now are cements with minimal adhesive properties, which bond surfaces together by interlocking into pores and imperfections in the surfaces of the underlying bone. KRYPTONITE™ Bone Cement represents a major advance in treating cranial defects due to trauma and surgery and it is the first non-toxic, low exotherm cement and bone void filler approved for cranioplasty applications that offers strong adhesive properties to organic and inorganic materials. KRYPTONITE™ Bone Cement is a selfsetting bone cement formed by combining three naturally occurring components to create an adhesive, non-toxic bone repair solution that cures with porous properties and is engineered to mimic natural bone. However, as mentioned above, KRYPTONITE™ should not be used in sternal closure cases, since its crosslinking speed is too high. Within just six minutes it will reach high adhesion strength. It has been used in some cases and provides an improvement over steel wire and pins, however a more appropriate alternative is still needed. The high strength which the cement reaches within a short time means it is not recommended for patients at risk of internal bleeding or other post surgical complications as use of this adhesive would delay the emergency surgery. These complications are likely to arise within 24 hours of the operation. Also this bone cement is used in conjunction with the traditional method of stainless steel wires, which still requires a second surgery for removal.

### Object of the Invention

It is an object of the present invention to provide a bio-compatible topical skin adhesive with the following qualities: good biocompatibility, good wet adhesion, relatively strong strength within 15 mins of application.

It is an object of the present invention to provide a bio-re-absorbable bone adhesive with the following qualities: good biocompatibility, good wet adhesion, degradation tailored to promote healing, initially low strength within 24 hours of application. It is a further objection of the invention to provide an adhesive for sternal closure that does not have a high bonding strength within 24 hours for emergency treatment convenience, but it should be enhanced over time, up to a certain strength to resist fracture after 1-2 days and reach a very high strength in a week's time. Degradation should be inversely proportional to healing within 3-4 months.

It is an object of the invention to provide a suitable, biodegradable bone adhesive tailored to the specific requirements of this application that can allow for easy access within 24 hours of the operation.

### Statements of the Invention

Accordingly to the present invention as set out in the appended claims, there is provided a dendritic or hyperbranched polymer comprising a plurality of branches, wherein at least one of the branches comprises at least one catechol moiety that is oxidisible to a semiquinone moiety and/or a quinone moiety, characterised in that the polymer further comprises at least one other functional group selected from the group consisting of: a vinyl functionality which may an unsubstituted vinyl or a functionalized vinyl group, an amine functionality, a disulfide functionality, at least one ionisable amine, diamine, tri-amine, amino alcohol functionality, and/or combinations thereof and wherein optionally at least one of the plurality of polymeric branches terminates with a hydroxyl functionality or terminates with a halide selected from the group consisting of: F, Cl, Br and I.

It is preferred that the dendritic or hyperbranched polymer of the invention is bioresorbable, biodegradable, and/or bioabsorbable.

Suitably, in the dendritic or hyperbranched polymer of the invention the catechol moiety is derived from a DOPA moiety or dopamine moiety or substituted phenethylamine. It will be understood that a DOPA moiety is one based on dihydroxyphenylalanine (DOPA), which can be unsubstituted or substituted with at least one other functional group. It will also be understood that a dopamine moiety means dopamine **(DA)** that can be unsubstitued or substituted with at least one other functional groups, such as amine groups.

Desirbly, in the dendritic or hyperbranched polymer of the invention, each of the plurality of polymeric branches may comprise an alkyl based chain from which further alkyl based chains split off to form further branches.

Suitably, in the dendritic or hyperbranched polymer of the invention, the dendritic or hyperbranched polymer comprises at least one other functional group.

Suitably, the at least one other functional group is selected from the group consisting of: a vinyl functionality which may an unsubstituted vinyl or a functionalized vinyl group, an amine functionality, a disulfide functionality, at least one ionisable amine, diamine, tri-amine, amino alcohol functionality, and/or combinations thereof. Desirable, the amine group may be a primary, a secondary or a tertiary amine group or a diamine group of general formula -NH-alkyl-NH₂, wherein the alkyl group is a linear or branched alkyl group having from 1 to 6 carbon atoms, and which can be substituted or unsubstituted with C₁₋₆ alkyl. Desirably, the amine group or diamine groups comprise cationic amine groups or amine groups that become cationic under certain conditions, i.e., ionisable. Suitably such conditions include physiological conditions. Particularly preferred are tertiary amines, as they are easily protonated at acidic pH.

In a particularly preferred embodiment, in the polymer of the invention the terminating vinyl group may be provided as a methacrylate group which can be unsubstituted or substituted with C₁₋₆ alkyl. The vinyl group may also be provided as C₁ - C₆ alkylmethacrylate, for example, methyl, ethyl propyl, butyl, pentyl or hexyl methacrylate group. The alkyl groups in these instances may be substituted or unsubstituted with at least one methyl or ethyl group.

Preferably, at least one co-vinyl monomers or trimer may be used in the process of the invention for copolymerisation with the catechol moiety and may have the general structure: 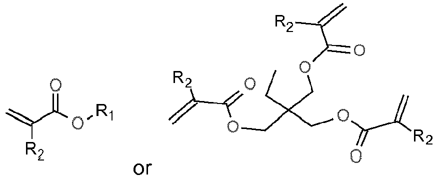 wherein R₁ is C₁- C₆ alkyl-amine selected from the group consisting of: C₀- C₆ alkyl-NH₂, C₀- C₆ alkyl-NHR₂, C₀- C₆ alkyl-NR₂ R₃, wherein independently R₂ and R₃ are -H or C₀- C₆ alkyl, and R₂ is C₀ - C₆ alkyl. Particularly preferred alkyl groups are methyl, ethyl, propyl or butyl groups, which can be linear or branched. A preferred alkyl group is methyl. Preferably, R₁ is methyl, ethyl or propyl, or combinations thereof. Preferably, the amine is dimethyl or diethyl amine. Trimer type starting materials for copolymerisation are useful where dendrimers are required, whereas monomers are used where hyperbranched polymers are desired.

Suitably, particularly preferred monomer are: poly(ethylene glycol) methacrylate, N-(2-hydroxypropyl)methacrylamide., 2-dimethylaminoethyl prop-2-enoate, 2(dimethylamino ethyl) methacrylate or 2(dimethyleamino ethyl) acrylate, although other monomers having vinyl and amine groups can be used. Preferably the trimer is trimethylol-propane triacrylate (TMPTA) or Pentaerythritol triacrylate and the dimer is Poly(ethylene glycol) diacrylate, ethylene glycol diacrylate and Diethylene glycol diacrylate

Suitably, the ratios of divinyl disulfide monomer or trimer to co-vinyl monomer may be in the range of from 1:99 to 99:1. A preferred polymer with some knotting has a ration of 10:90.

In a preferred embodiment, in the dendritic or hyperbranched polymer of the invention, at least one of the plurality of polymeric branches terminates with a hydroxyl functionality. Desirably, at least one of the each of the plurality of polymeric branches terminates with a halide selected from the group consisting of: F, Cl, Br and I.

Suitably, the dendritic or hyperbranched polymer of the invention is polydisperse and has a relative high polydispersity index meaning that there will be a lot of molecular weight enhancement when go crosslinking as determined by gel permeation chromatography (GPC).

In a preferred embodiment, the alkyl based chain of the polymer may be an ethylene chain which may be unsubstituted or substituted with a linear or branched C₁₋₆ alkyl. Unsubstituted ethylene chains are particularly preferred.

In a particularly preferred embodiment, the polymer of the invention is hyperbranched and comprises a plurality of branched alkylene chains, wherein at least one of the alkylene branches comprises at least one DOPA moiety or dopamine moiety in the alkylene chain, and the alkylene sidechain is further substituted with at least one vinyl group or vinyl functionality; and wherein at least one of the alkylene branches terminates with at least one of a carbocation, a halide, an ionisable amine, diamine and a hydroxyl group.

In a preferred embodiment there is provided a polymer comprising at least one polymeric ethylene backbone having: at least one first sidechain repeating unit, wherein one end of each sidechain is covalently bonded to the ethylene backbone by an ester, amide or thioate linkage, and wherein the other end of each first sidechain comprises a catechol moiety, such as DOPA moiety or dopamine moiety and at least one of a vinyl group, a hydroxide, a halide, an amine group or a diamine group to form a polymer or comprises at least one an ester, amide or thioate linkage to a polymeric ethylene backbone (which can be same or different) to form a branched polymer having at least two polymeric ethylene backbones, and wherein each of the first sidechains further comprises at least one disulfide group located between either ends of the first sidechain repeating unit; and at least one second sidechain repeating unit, wherein one end of each sidechain is covalently bonded to the at least one polymer ethylene backbone by an ester, amide or thioate linkage, and wherein the other end of each second sidechain comprises with an ionisable amine or a diamine group.

In a preferred embodiment, the polymer further comprises a plurality of additional polymer backbone chains having the at least one first sidechain repeating unit and the at least one second sidechain repeating unit as defined above, the plurality of additional polymer backbone chains being arranged in a branched polymeric repeating pattern to form a hyperbranched polymer.

Thus the invention provides a linear, branched or hyperbranched polymer having controlled molecular weights and narrow molecular weight distributions. Suitably, the molecular weight, and the composition (i.e. the number and ratio) of first and second sidearm chains can be controlled or adjusted by appropriate choice of the co-monomer composition used to provide the polymer (for example, by a Michael Addition, click chemistry, RAFT, ATRP or DE-ATRP or other controlled living polymerization process). Desirably, quaternisation reaction of the polymer with reagents such as methyl iodide can leads to water-soluble cationic polyelectrolytes with linear, branched or hyperbranched structures. The polymers of the invention are a significant achievement, as it is known in the art that synthesis of well-defined branched polymers with ionic or ionisable groups is difficult.

Preferably, the polymer of the invention is a knotted polymer which can be single cyclized or multi-cyclized.

Desirably, in the dendritic or hyperbranched polymer of the invention the vinyl group or vinyl functionality is a methacrylate group which can be unsubstituted or substituted with a linear or branched C₁₋₆ alkyl. Methyl and ethyl substituents are preferred.

Suitably, at least one of the plurality of polymeric branches comprises at least one of an ester, ether, thioate or amide functionality.

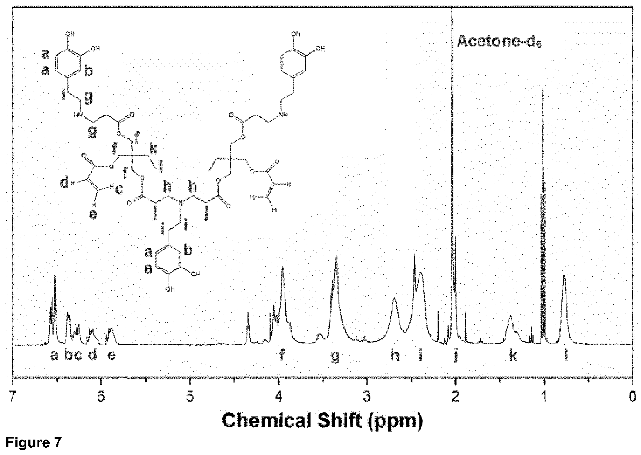

In a particularly preferred embodiment, of the invention, there is provided a dendritic or hyperbranched polymer having the ¹H NMR spectrum shown in Figure 7.

Suitably, the polymer of the invention may be derived from co-polymerisation of dopamine HCl monomer and trimethylol propane triacrylate monomer (TMPTA).

Suitably, the polymer is poly-dopamine-co-acrylate.

In a related aspect of the invention, in the dendritic or hyperbranched polymer of the invention, at least one of the plurality of branches may be crosslinked with a crosslinking agent or crosslinker system, which can optionally be used with a crosslinking promoter. Suitably, the branches may be crosslinked with chitosan or a crosslinker system that is promoted by oxidation. Desirably, the crosslinker system is permanganate, Fe(III), fibrinogen, HPR and peroxide, or combinations thereof. The Fe(III) may be provided in the form of FeCl₃, Fe(acaC)₃ or other Fe(III) salt.

In a related aspect still, the polymer of the invention or a composition of a polymer of the invention may be used as medical adhesive or bone cement. Suitably, the medical adhesive is an adhesive for skin incision and/or internal wound closure. In a related embodiment, the bone cement is a cement for sternum bone closure or other orthopaedic operating procedure. Accordingly, the polymer of the invention may be used in the manufacture of a medical adhesive or a bone cement.

In a further related aspect still, there is provided a process for synthesising a dendritic or hyperbranched polymer comprising at least one catechol moiety by polymerising a divinyl monomer or a triacrylate with other optional polymerisable monomers and/or initiators including those having at least one catechol moiety under suitable polymerisation conditions to effect polymerisation of the monomer by a Michael Addition, a click chemistry or a living polymerisation process.

### Brief Description of the Drawings

Figure 1 shows synthetic mechanism and strategy for the catechol-modified dendritic polymer adhesive of the invention. The target polymeric adhesive was prepared via a one step Michael addition or click chemistry procedure;
Figure 2 shows synthesis route and schematic structure of poly-dopamine-co-triacrylate, Michael addition;
Figure 3 shows crosslinking mechanism of PDA with Fe³: (a) cohesive coupling by oxidized DOPA (b) one ferric ion centers in three DOPA residues by complexation (c) redox radical polymerization.;
Figure 4 shows the Redox control and the stepwise adsorption and cross-linking of DOPA residues with multi-thiol groups (fibrinogen) as crosslinker. (a) The oxidation of unadsorbed dopa to dopa-quinone by disulfide bond in fibrinogen. (b) Cohesive coupling of reducing thiolates with dopa-quinone by Michael addition. (c) Adhesive binding with inorganic surfaces via H-bonding or coordination. (d) Adhesive binding with organic surfaces via Michael addition or Schiff reaction.
Figure 5 shows a schematic representation of PDA crosslinking via HRP and H₂O₂;
Figure 6 shows a GPC trace for the purified poly-dopamine-co-acrylate (PDA);
Figure 7 shows ¹H NMR trace of poly-dopamine-co-acrylate (PDA);

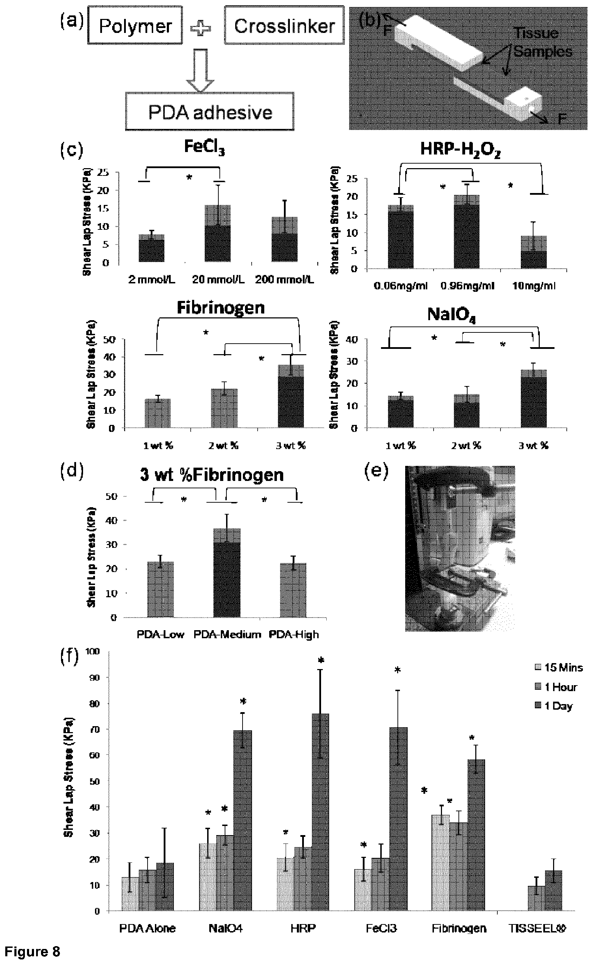

Figure 8 shows (a) Composition of PDA adhesive (b) 3D animation of wound closure strength test produced using Autodesk Inventor(c)Shear Lap tests results of PDA-Medium polymer working with four different curing agents in different concentrations after curing time of 15 mins on porcine skin samples (d) Shear Lap tests results of PDA-Low, PDA-Medium and PDA-High polymer working with the optimized concentration of fibrinogen(3 wt %) after curing time of 15 mins (e) Photo showing how the shear Lap tests were done (f) Shear Lap tests results of PDA-Medium polymer working with four different curing agents in optimised concentrations after curing time of 15 mins, 1hour and 1day controlled with commercially fibrin glue TISSEEL^{®}. The results indicate fibrin glue is very weak and is not strong even enough to be tested within 1 h curing time. The poly-dopamine-co-acrylate (PDA) of the invention can reach a much higher adhesion strength in a short time (15 mins);

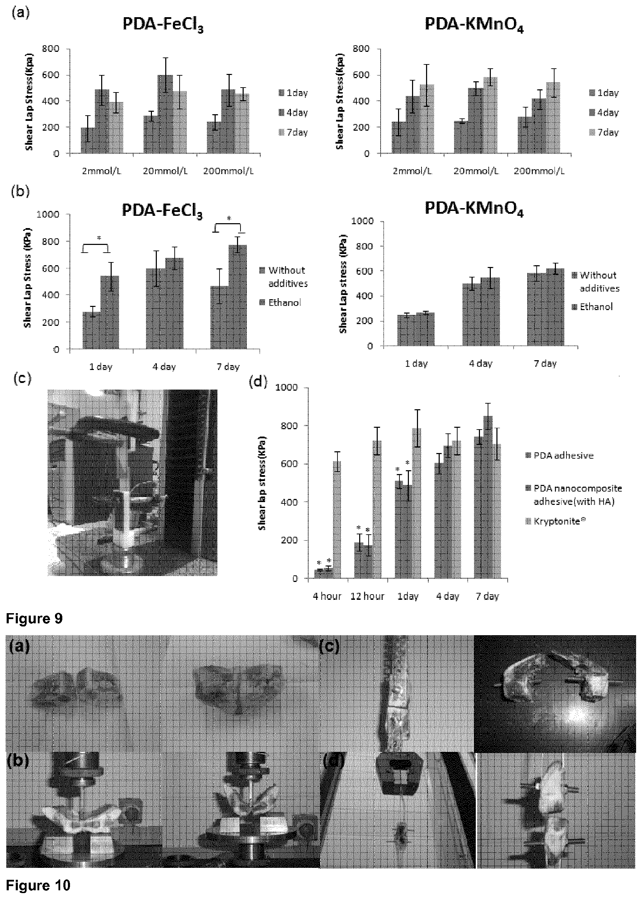

Figure 9 shows (a) preliminary PMMA shear lap tests results of PDA polymer working with two different curing agents in low/medium/high concentrations after curing time of 1 day, 4 days and 7 days. (b) Shear lap tests results of PDA polymer working with the optimized concentration of (20 mmol/L) curing agents and the assistance of different additives (ethanol and chitosan) after a curing time of 4hs, 12h, 1 day and 7day. (c) Photo of shear-lap test on PMMA sheets (d) Shear Lap tests results of PDA adhesive and PDA nanocomposite adhesive (with 10% HA) working in optimized curing conditions after curing time of 4hs, 12hs, 1 day, 4 days and 7days compared to commercially bone cements KRYPTONITE™. * indicates statistically significant differences between groups or from KRYPTONITE™ group (ANOVA), n=5. The results indicate Kryptonite™ hardens immediately (strength>900 KPa) which could delay emergency operation in the case of post-operative complications. The strength of the poly-dopamine-co-acrylate (PDA) adhesive of the invention was gradually increases with time and finally reach 760 KPa at 7 days;
Figure 10 shows (a) the wet sternal bone samples are cut ready and then glued for testing. (b) Compression tests ongoing and finished. (c) Bone samples are drilled and clamped using metal strips for lateral distraction tests. (d) Lateral distraction tests ongoing and finished.
Figure 11 shows adhesion strength was tested by loading (a) compression and (b) lateral distraction force on sternal halves bonding with PDA adhesive after a curing time of 1 day, 4 days or 7 days controlled with commercially bone cements KRYPTONITE™.
Figure 12 shows (a)PDA polymer can degrade hydrolytically by breaking the ester bond (R-triacrylate monomer, R'- dopamine monomer) (b)PDA polymer can degrade to almost 60 wt% in PBS buffer solution at 37°C over one month time.
Figure 13 shows 3T3 fibroblast viability analysis in the presence of 200 µg PDA with four different crosslinkers in comparison to PDA alone and TISSEEL^{®} fibrin glue. At 7 days, viability is not significantly reduced in the presence of PDA relatively to cells alone. * P<0.05.

### Detailed Description of the Invention

The invention pertains to the development of an adhesive for application in repairing the sternum following a median sternotomy and also for topical skin closures or for use in other orthopaedic operating treatments, such as shoulder, elbow, spine, hip, knee, wrist, foot and ankle surgery. The inventors have developed a catechol modified dendritic polymer, which achieves this goal. The adhesive of the invention is biocompatible and safe for internal use. It has wet adhesive qualities. It has a controllable curing speed with the help of different crosslinkers to allow for emergency operation within the first 24 hours and also skin closure in a short time (15 mins). Using crosslinkers (KMnO₄, Fe³⁺, HRP&H₂O₂ and fibrinogen) the crosslink rate can be tailored, as well as a number of other qualities, to achieve this. For sternum bone closure, after the first couple of days the strength of the adhesive must increase to securely fixate the sternum for repair, and must achieve this strength without excessive heat production, which would cause necrosis to the surrounding tissue. Following the healing period the adhesive must degrade into non-toxic/carcinogenic/teratogenic by products that can be safely metabolised in the body.

Preliminary tests performed have already shown that for skin closure, the adhesives displays high adhesion strength in 15mins which is enough to close the wound. For sternum bone closure, the polymerisation rate over the first few days can be controlled and the adhesive of the invention shows excellent adhesive properties to the bone samples. The by-products of the degradation process of the adhesive of the invention polymer also fulfil the biocompatible necessities outlined earlier.

There are many requirements for a medical bone adhesive to clinical application in trauma and orthopaedic surgery: The adhesive and its degradation products must be non-toxic, non-carcinogenic, and non-teratogenic. The adhesive must be biocompatible to bone and surrounding tissue (no tissue irritation or tissue necrosis) with minimal heat development during polymerization. It should be biodegradable in a prefixed time - by degradation and cellular resorption - without acting as a barrier to callus formation (osteogenesis). The degradation process should act inversely proportional to healing, to ensure mechanical stability. The adhesive must bond in moist environment (high bonding strength in situ) to allow early weight bearing, needs sufficient elasticity to ensure stability to tensile strength. Easy preparation, practicability and applicability, minimal compression of volume, stability during storage, sterility and efficiency of bone adhesive. For this specific application the adhesive will also need an initially slow polymerization to allow for quick access in case of emergency operation within the first 24 hours.

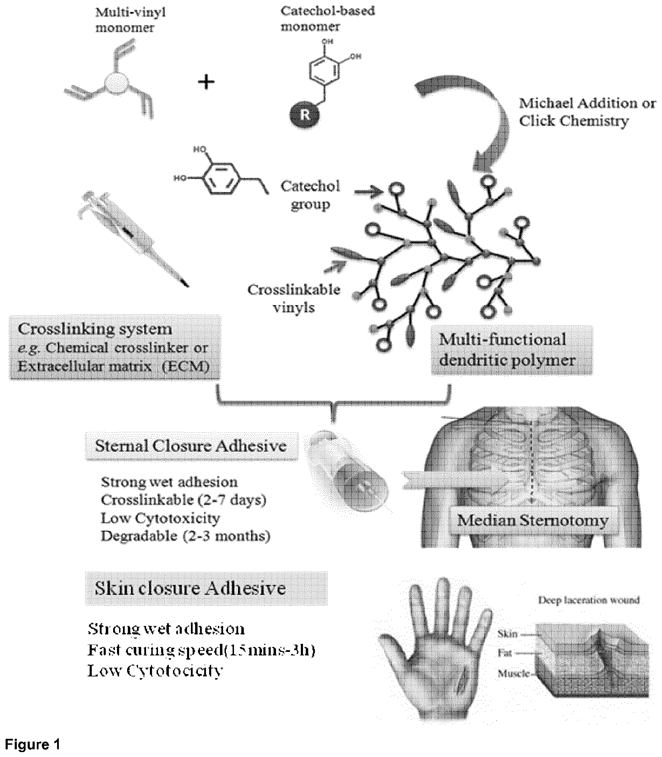

This invention focuses on developing catechol-modified dendritic polymer adhesives to overcome the drawbacks of current tissue adhesives using their unique structures and functionalities (Figure 1). This strategy has been successfully applied on incisional skin wounds in the inventors group and has shown very promising results. The successful outcome will result in major advances in healthcare - most notably for treatment of internal wound and surgical incisions wound, but also for other wound healing treatments in the future. These exciting results lead us to extend this new approach for bone adhesive application. The preliminary test results demonstrated that the pilot catechol-modified polymer that was developed showed excellent adhesion and mechanical properties for the bone model. Even more exciting is the adhesive strength can be controlled within the first week to allow easy access within the first day of bonding and gaining strength throughout the week. PDA was selected for this project as it contains dopa structure, which allows for wet adhesion, and also the functional groups present in PDA allow for tailoring of the mechanical properties.

In this study, the development of novel biodegradable and crosslinkable dendritic polymers, poly-dopamine-co-acrylate (PDA) was targeted as wet tissue adhesive materials, for example, adhesives for internal use. PDA can easily crosslink with a variety of different crosslinkers, so that the adhesive of the invention can either work as strong, secure and fast curing adhesive for topical wound closure or as controllable curing adhesives for sternum bone closure cases.

Our objective was to mimic MAPs by way of dopamine inclusion (derivative of L-3,4-dihydroxyphenylalanine DOPA, an abundant amino acid in MAPs) within a hyperbranched structured polymer to form an adhesive. Thus, the present inventors have designed and synthesized bio-inspired dendritic polymers for wet tissue adhesive with superior properties over the current tissue adhesives by using state of the art Deactivation-Enhanced ATRP (DE-ATRP) synthetic approach.

### Synthesis of poly-dopamine-co-acrylate PDA

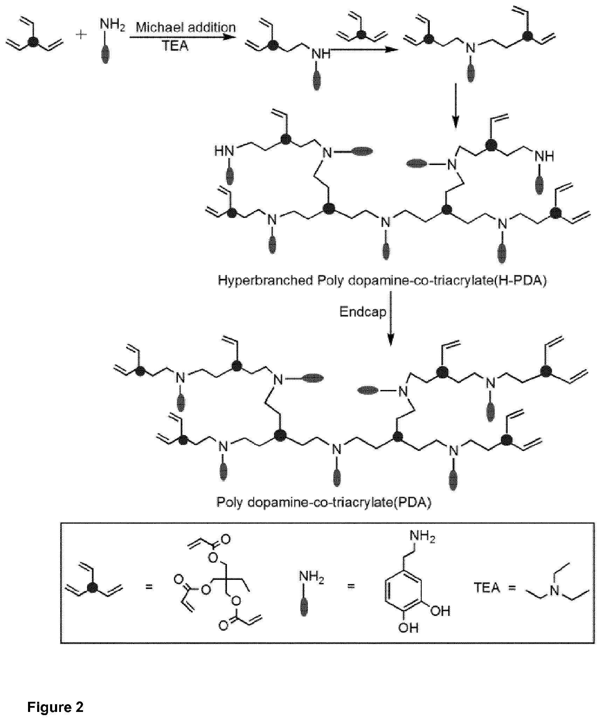

Dendritic/hyperbranched polymer poly-dopamine-co-acrylate (PDA) was successfully synthesised through the self-condensation of dopamine and trimethylol-propane triacrylate (TMPTA) monomer by Michael additions to obtain high molecular weight polymers (around 90.0kDa) (Figure 2). The targeted dendritic polymers poly-dopamine-co-acrylate (PDA) consists of catechol, vinyl and hydroxyl functional groups, leading to tailorable functionality, crosslinking (with crosslinker), biodegradability, low cytotoxicity and desired mechanical properties on dry/wet tissue.

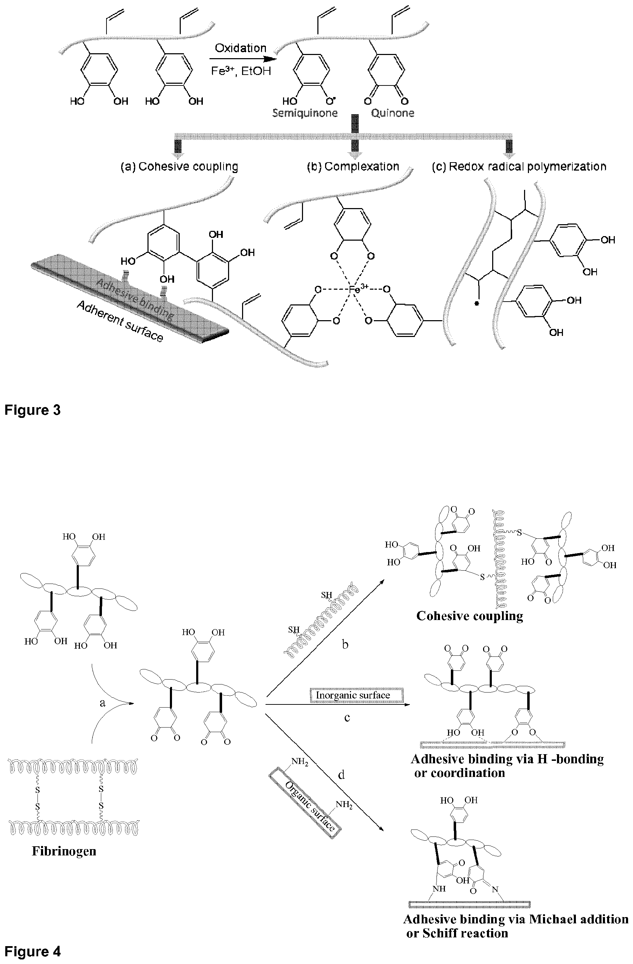

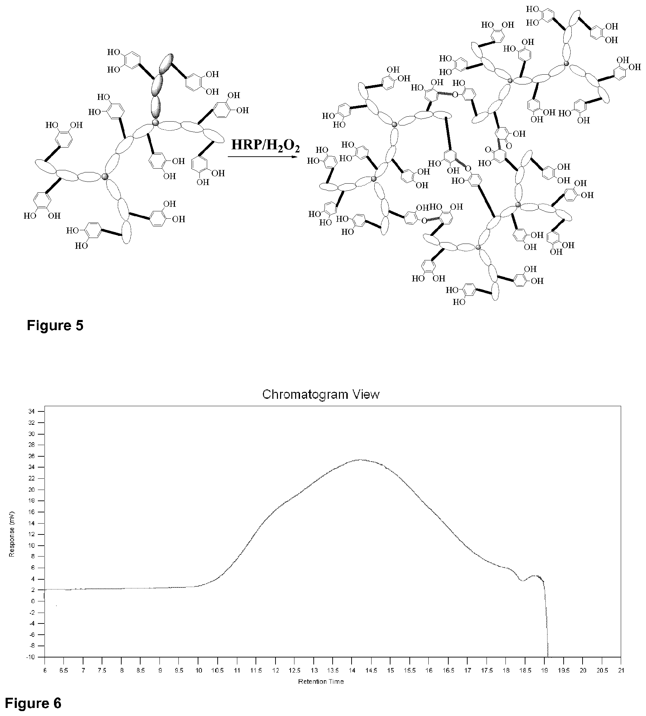

To control the crosslinking speed it is important to understand how catechol/dopamine adhesive systems achieves adhesion. Transition metals play an integral role in the generation of a non-crystalline biological material. Oxidation of dopamine by one electron to a semiquinone occurs at a potential similar to Fe³⁺ to Fe²⁺ reduction. Semiquinone is a kind of radical which can bring about polymer-polymer coupling for cohesive bonding. It is believes that the radicals also couple directly to surfaces for adhesive interactions. Furthermore, quinone can chelate with iron to lead another way of crosslinking. We propose that the iron centre in cross-link three DOPA residues (Figure 3). With these latest observations, it appears that dopamine plays two roles in catechol/dopamine adhesive systems. Adhesive bonding to surfaces is derived from the reduced form. Cohesion in the bulk is provided by the one-electron (semiquinone) or two-electron (quinone) oxidized forms of dopamine. PDA has a tailored crosslinking speed by utilising crosslinker (KMnO₄, Fe³⁺, HRP/H₂O₂ or denatured fibrinogen). When using NaIO₄, a kind of strong oxidant as curing agent DOPA is immediately converted to DOPA-quinone which triggers intermolecular crosslinking, promoting the cohesive strength of PDA polymer. The reaction is relatively rapid although some of the adhesion strength might be compromised. The use of periodate (NaIO₄) to crosslink PDA polymer might be a potential source of hidden peril for clinical use in consideration of its oxidizing properties. However, as we know, sodium periodate is widely used for clinical use for its oxidizing properties, for example, it is used to oxidize cellulose and create a biocompatible and biodegradable compound that can be used as suture, as a scaffold for tissue engineering, or for drug delivery. Consumption of electrons in DOPA by an oxidation reaction will be accompanied by the acquisition of electrons in periodate ion to form reduction product iodate ion (IO₃⁻) an additive in salt, which can be further reduced to iodide ion (I⁻) which is non-toxic. Ethanol is also involved in the crosslinking system when the crosslinker is oxidative as Fe³⁺/ C₂H₅OH or NaIO₄ / C₂H₅OH is a redox pair which can generate radicals in aqueous solution which can react with the vinyl groups in PDA and thus speed up crosslinking speed. Compared with other strong oxidative crosslinkers such as NaIO4, fibrinogen is better at achieving a relatively higher adhesion strength in a short curing time as thiols resulting from the scission of S-S bond can couple with DOPA⁻ quinone formed by DOPA oxidation which is crucial for improving cohesion strength among the adhesive polymer as well as providing an anti-oxidant environment which is beneficial for preserving adhesion strength. As depicted in Fig.4, the redox systems of disulfide and thiolates may be efficiently adapted to be firstly an antioxidant (Fig.4 a and b) which ensures enough adhesion strength in the initial stage. Later the disulfide and thiolates act as a cross-linking partner for DOPA-quinone which is crucial for improving cohesion strength among the adhesive polymer (Fig.4c). HRP is a hemoprotein that enables cross-linking between phenol and aniline derivatives with decomposed H₂O₂ in the presence of phenolic hydroxyl groups8. It has been proved that the HRP concentration affects the crosslinking kinetics, while the variation in the H₂O₂ concentration primarily affects the mechanical properties. From our tests, HRP in H₂O₂ is a non-cytotoxic curing agent for PDA which can achieve a high adhesion strength (76 ±13.4 KPa) after one day curing time. We propose the crosslinking mechanism of PDA with HRP-H₂O₂ in Fig. 5.

### Poly-dopomine-co-ocrylate(PDA) synthesis - Fabrication protocol for Poly Dopamine-co-acrylate(PDA)

Trimethylolpropane triacrylate monomer(2.96 g, 10 mmol), Dopamine hydrochloride monomer(1.90g, 10 mmol) and DMSO(10 g) were added into the two neck flask. 60µL inhibitor(4-Methoxy-benzaldehyde) was added into the mixture to get rid of the possibility of free radical polymerization.The mixtures were stirred until a clear solution formed. Oxygen was removed by bubbling argon through the solution for 10 mins at room temperature. TEA (1.42 g, 14 mmol) was added at last when going through bubbling argon and magnetically stirred for 5 mins more. The solution was stirred at 600 rpm and the polymerization was conducted at 60deg C for 2 h in an oil bath. Take GPC sample every one hour.

### Purification protocol for Poly Dopamine-co-acrylate(PDA)

40 ml THF was added into the polymer mixture and then magnetically stirred it until it was completely blended. As the TEA saline is insoluble in THF, the mixture solution went cloudy. The surplus TEA saline was separated by centrifugalization(5 mins, 4000 rpm). The clear polymer solution was added dropwise into a large amount of precipitation agent (hexane:Ethanol = 1: 1) (500 mL) with 100 µL inhibitor(4- Methoxy-benzaldehyde) under magnetically stirring condition. The precipitate system was left still for 0.5 h and then the upper clear liquid was poured out. Acetone was used to dissolve the polymer at the bottom of the beaker to make a 50 wt% solution.

### GPC results of Poly Dopamine-co-acrylate(PDA)

The GPC trace for the purified Poly Dopamine-co-acrylate (PDA) shown in Figure 6.

### Optimising Crosslinker

For each tissue adhesion experiment, PDA was first dissolved in acetone to a concentration of 50 wt% by vortexing for 1 min. When using FeCl₃ and NaIO₄ as a crosslinker, 60µl 2mmol/L, 20 mmol/L and 200mmol/L FeCl₃/H₂O or 1 wt%, 2 wt% and 3 wt% NaIO₄/H₂O were mixed with 60µl of pure ethanol in an eppendorf tube. When using fibrinogen as a curing agent, fibrinogen were dissolved in 0.9% saline water to a 1 wt%, 2 wt% and 3 wt% concentration and 60µl solution was used for each test. PDA adhesive was prepared by mixing 300µl 50 wt% PDA solution with crosslinker solution and then the fully mixed adhesive fluid was applied at room temperature to both surfaces of porcine skin tissue substrates which were put in PBS buffer solutions before tests. For the enzyme mediated oxidative crosslinker, 20µl 0.1 wt%, 0.2wt% and 0.3wt% horseradish peroxide were mixed with PDA solution before 20µl 0.1 wt%, 0.2wt% and 0.3wt% H₂O₂ were added and gently vortexed to ensure homogeneous mixing. Control specimens with no crosslinker were treated in the same manner as the adhesive samples. The two skin pieces were then overlapped ensuring that the proper overlap area (2 cm x 1.5cm) was maintained. Gauze saturated with PBS was wrapped around the samples to maintain hydration throughout the duration of the curing time. The samples were left curing at room temperature for a period of 15 mins, 1 hour, and 24 hours before strength testing.

For comparison purposes, tissue surfaces were adhered together using a commercial fibrin surgical sealant (TISSEEL^{®}, Baxter) which was prepared according to manufacturer's instructions. Fibrinogen was mixed with aprotonin solution then thrombin was combined with a calcium chloride solution. The two mixtures were then loaded into a set of duplex syringes. As they were dispensed, the two components were mixed at the outlet of the syringe system where they cross-linked to form the fibrin adhesive. 300µL of fibrin was applied in the same manner as the PDA adhesive samples.

### Mechanical Test for PDA -Qualifying adhesion strength on porcine skin

The tissue adhesive properties of PDA polymer with four different curing agents were studied on porcine dermal skin surfaces after curing the adhesive for 15 mins, 1 hour and 1 day at room temperature. The concentration of curing agents was optimised by carrying out shear lap tests on PDA-Medium polymer with four curing agents in low/medium/high concentrations after curing time of 15 mins (Fig. 8(c)). The molecular weight of PDA polymer was optimised by carrying out shear lap tests on PDA-Low, PDA-Medium and PDA-High polymers with the optimized concentration of fibrinogen (3 wt %) after a curing time of 15 mins (Fig. 8(d)). The tissue adhesive properties of PDA-Medium polymer (optimised) with optimised concentration of four curing agents were studied on porcine dermal skin surfaces after curing the adhesive for 15 mins, 1 hour and 1 day at room temperature. Control experiments were carried out on PDA polymer without curing agent and TISSEEL® fibrin glue (Fig. 8(f)). Fibrin glue showed no signs of adhesion in the 15 minutes of curing time as all specimens failed before or during attempts to load into the mechanical test machine. The fibrin adhesive yielded a mean lap shear strength of 9.45 KPa (SD = 3.3 KPa, n = 5) and 15.34 KPa (SD = 4.65 KPa, n = 5) after 1hour and 1day curing time respectively (Fig. 8(f)), which are close to the strength reported by others for fibrin adhesives8,9. Failure of the fibrin glue bound samples was noted to be of the cohesive type.

From our primary investigation, the maximum adhesion force reached a plateau value after 15 mins in a short time and increased to around 70 KPa after 1day curing time. Especially high adhesion strength (37±5.6KPa) can be formed in just 15mins which is substantial for incision skin wound closure when fibrinogen was used as curing agent. From our test results, fibrinogen was the best for achieving a relatively higher adhesion strength in a short time and HRP was the best for achieving a high adhesion strength (76 ±13.4 KPa) after a one day curing period.

### Qualifying adhesion strength on PMMA sheets.

Shear-Lap stress measurements were performed on PMMA sheets to get the longitudinal shear modulus following the procedures described in ASTM standard F2255-05. Slight modifications to the protocol were used where appropriate. Samples of PMMA measuring 60 mm x15 mm x 3 mm were used as bone substitute for shear lap tests. A Zwick tensile tester with a 1000 N load cell was used, which comprised of a fixed member that carried one substrate holder and a movable member that carried the loading cell, two compatible holders which fixed the test specimens between two members and a driving mechanism that allowed the moving member to move at a constant rate as required. Nylon was selected as the material for use as the substrate holders. Nylon provided sufficient strength to withstand the shear stresses imposed on the piece. G-clamps were used to fix the samples onto the holders. The bonded fixtures were loaded until failure at a cross-head speed of 5 mm/min. A minimum of five samples per group were tested using this approach.

Ex-vivo mechanical strength measurements: The Instron 4467 materials testing systems were used both in compression and lateral distraction testing. The adhesion strength at failure by loading compression and lateral distraction strength were measured with lamb sternal bone specimens bonded while wet. Each sternum was cut vertically in half as in a median sternotomy using a band-saw and then was cut horizontally into 10 samples. After cutting the samples, 5 pairs of samples were tied together with elastic bands and placed in PBS buffer for 24 hours.

Compression measurements were performed on lamb sternal bone following the procedures described in ASTM standard D905 - 08e1 with modifications. The machine had interchangeable clamps to allow for compression tests. A compressor was chosen specifically for this test to focus the applied force on the adhesion site. This would ensure the most accurate results from the experiment. Before the experiment the bonding samples were mounted on two blocks to prevent the compressor from just crushing the bone and to ensure that the results obtained were for the strength of the adhesion at the site of the cut. A third block was used to keep the space between the mounting blocks consistent for all experiments. The bonding site was carefully aligned with the centre of the compressor. The rate of the crosshead movement was 5 mm/min. After testing, the bonding area of the bone at the site of adhesion was measured.

The lateral distraction tests were performed on lamb sternal bones following the procedures described in ASTM standard D2095-96 with modifications. The bones were drilled through using a 3 mm drill bit and hand drill. The hole was drilled a little back from the adhesion site to prevent damaging the surface area. After drilling, the samples were glued and left curing for certain periods, then the bonding samples were clamped sideways in the machine by a pair of vices which move at a constant rate of 5 mm/min in the opposite direction. A metal strip with holes was used to fix the bones to the vice. The metal strip was held to the bone using a 3 mm threaded bar and nuts. The ends of the metal strip that were not connected to the bone were clamped in the vices. For some of the irregular samples it was necessary to cut off the excess meat to ensure the samples were straight and could be placed in the machine without placing an initial pressure on the adhesion. After testing each sample the metal strip and bar was removed and replaced each time and a new sample was tested. After each test the bonding area was measured and the lateral distraction strength was calculated from this using the standard formula.

The adhesive strength of the nanocomposites was investigated by shear-lap tests (in-vitro longitudinal shear) on two PMMA sheets as bone substitute, bonded together because of the difficulty in obtaining bone samples of the appropriate geometry and size (Figure 9(c)). The concentration of curing agents was optimized by carrying out shear lap tests on PDA polymer with two curing agents (FeCl₃ and KMnO₄) in low/medium/high concentrations (2 mmol/L, 20 mmol/L and 200 mmol/L) (Figure 9(a)). After the best concentration for each curing agent was decided upon (20 mmol/L), the curing condition was optimized by carrying out shear lap tests on the PDA polymer with the two optimized curing agents and also ethanol as additive (Figure 9(b)). Lastly, the optimized PDA adhesive (20 mmol/L FeCl₃/H₂O+10µl ethanol) and PDA nanocomposite adhesive (PDA adhesive+10wt% HA) were analyzed on PMMA sheets in comparison to KRYPTONITE™ (Figure 9(d)). From our investigation, at the early stage (≤1 day) KRYPTONITE™ samples showed almost full strength, yielding a mean lap shear strength of 614 KPa (SD = 50 KPa, n = 5) and 787 KPa (SD = 96 KPa, n = 5) after 4 hours and 1day curing time respectively (Figure 9(d)). Such a high lap shear strength would require the surgeon to use an electrical saw if emergency re-entry was required, putting the patient at elevated risk. However, PDA adhesive has a controllable curing speed, allowing setting to take place over one week due to its unique structures and functionalities. When curing with FeCl₃, a much lower relative shear strength (509 KPa, SD = 37 KPa, n = 5) can be achieved within one day, for emergency convenience, but eventually forming a high shear strength of 743 KPa (SD = 39 KPa, n = 5) after 7 days curing which is substantial for sternal wound closure. The PDA adhesive with 10% HA reinforcement showed significant improvement to around 850 KPa (SD = 70 KPa, n = 5) after 7 days curing (Figure 9(d)). The increment in the adhesive strength is due to the strong hydrogen bonding interactions of large numbers of end functionality (catechol groups) of the hyperbranched polymer along with polar groups in the HA systems with the hydroxyl groups on the adherent substrate (Figure 9).

Ex-vivo compression (Figure 10(a) and (b)) and lateral distraction measurements (Figure 10(c) and (b)) yielded the mechanical strength by loading compression and lateral distraction on PDA nanocomposite adhesive bonding the two lamb sternal halves. After introducing HA particles into the hydrophobic PDA polymer, the adhesive is less fluid, making it better for sticking to the bone and not prematurely slipping away or penetrating into the bone pores. KRYPTONITE™ yielded a mean compression strength of 514 KPa (SD = 97 KPa, n = 5) which is nearly the full strength because of the fast curing rate (Figure 11(a)) and a mean lateral distraction strength of 255 KPa (SD = 57 KPa, n = 5) (Figure 11(b)) in 1 day curing time. However, PDA adhesive has a controllable curing speed within one week and a relatively lower adhesion strength in one day when loading compression (251 KPa, SD = 52 KPa, n = 5) (Figure 11(a)) and lateral distraction (171 KPa, SD = 45 KPa, n = 5) (Figure 11(b)) force but eventually increased to a high compression strength of 623 KPa (SD = 71 KPa, n = 5) (Figure 6(a)) and lateral distraction strength of 633 KPa (SD = 52 KPa, n = 5) (Figure 11(b)) after 7 days curing time which is substantial to withstand the stresses in-vivo. Upon completion of the test, all samples showed an adhesive failure with the bone surfaces, rather than cohesive failure. The tissue adhesive properties of PDA polymer with four different curing agents were studied on porcine dermal skin surfaces after curing the adhesive for 15 mins, 1 hour and 1 day at room temperature. The concentration of curing agents was optimised by carrying out shear lap tests on PDA-Medium polymer with four curing agents in low/medium/high concentrations after curing time of 15 mins (Fig. 8(c)). The molecular weight of PDA polymer was optimised by carrying out shear lap tests on PDA-Low, PDA-Medium and PDA-High polymers with the optimized concentration of fibrinogen (3 wt %) after a curing time of 15 mins (Fig. 8(d)). The tissue adhesive properties of PDA-Medium polymer (optimised) with optimised concentration of four curing agents were studied on porcine dermal skin surfaces after curing the adhesive for 15 mins, 1 hour and 1 day at room temperature. Control experiments were carried out on PDA polymer without curing agent and TISSEEL® fibrin glue (Fig. 8(f)). Fibrin glue showed no signs of adhesion in the 15 minutes of curing time as all specimens failed before or during attempts to load into the mechanical test machine. The fibrin adhesive yielded a mean lap shear strength of 9.45 KPa (SD = 3.3 KPa, n = 5) and 15.34 KPa (SD = 4.65 KPa, n = 5) after 1 hour and 1day curing time respectively (Fig. 8(f)), which are close to the strength reported by others for fibrin adhesives8,9. Failure of the fibrin glue bound samples was noted to be of the cohesive type.

From our primary investigation, the maximum adhesion force reached a plateau value after 15 mins in a short time and increased to around 70 KPa after 1day curing time. Especially high adhesion strength (37±5.6KPa) can be formed in just 15mins which is substantial for incision skin wound closure when fibrinogen was used as curing agent. From our test results, fibrinogen was the best for achieving a relatively higher adhesion strength in a short time and HRP was the best for achieving a high adhesion strength (76 ±13.4 KPa) after a one day curing period.

### Qualifying adhesion strength on PMMA sheets.

Shear-Lap stress measurements were performed on PMMA sheets to get the longitudinal shear modulus following the procedures described in ASTM standard F2255-05. Slight modifications to the protocol were used where appropriate. Samples of PMMA measuring 60 mm x15 mm x 3 mm were used as bone substitute for shear lap tests. A Zwick tensile tester with a 1000 N load cell was used, which comprised of a fixed member that carried one substrate holder and a movable member that carried the loading cell, two compatible holders which fixed the test specimens between two members and a driving mechanism that allowed the moving member to move at a constant rate as required. Nylon was selected as the material for use as the substrate holders. Nylon provided sufficient strength to withstand the shear stresses imposed on the piece. G-clamps were used to fix the samples onto the holders. The bonded fixtures were loaded until failure at a cross-head speed of 5 mm/min. A minimum of five samples per group were tested using this approach.

Ex-vivo mechanical strength measurements: The Instron 4467 materials testing systems were used both in compression and lateral distraction testing. The adhesion strength at failure by loading compression and lateral distraction strength were measured with lamb sternal bone specimens bonded while wet. Each sternum was cut vertically in half as in a median sternotomy using a band-saw and then was cut horizontally into 10 samples. After cutting the samples, 5 pairs of samples were tied together with elastic bands and placed in PBS buffer for 24 hours.

Compression measurements were performed on lamb sternal bone following the procedures described in ASTM standard D905 - 08e1 with modifications. The machine had interchangeable clamps to allow for compression tests. A compressor was chosen specifically for this test to focus the applied force on the adhesion site. This would ensure the most accurate results from the experiment. Before the experiment the bonding samples were mounted on two blocks to prevent the compressor from just crushing the bone and to ensure that the results obtained were for the strength of the adhesion at the site of the cut. A third block was used to keep the space between the mounting blocks consistent for all experiments. The bonding site was carefully aligned with the centre of the compressor. The rate of the crosshead movement was 5 mm/min. After testing, the bonding area of the bone at the site of adhesion was measured.

The lateral distraction tests were performed on lamb sternal bones following the procedures described in ASTM standard D2095-96 with modifications. The bones were drilled through using a 3 mm drill bit and hand drill. The hole was drilled a little back from the adhesion site to prevent damaging the surface area. After drilling, the samples were glued and left curing for certain periods, then the bonding samples were clamped sideways in the machine by a pair of vices which move at a constant rate of 5 mm/min in the opposite direction. A metal strip with holes was used to fix the bones to the vice. The metal strip was held to the bone using a 3 mm threaded bar and nuts. The ends of the metal strip that were not connected to the bone were clamped in the vices. For some of the irregular samples it was necessary to cut off the excess meat to ensure the samples were straight and could be placed in the machine without placing an initial pressure on the adhesion. After testing each sample the metal strip and bar was removed and replaced each time and a new sample was tested. After each test the bonding area was measured and the lateral distraction strength was calculated from this using the standard formula.

The adhesive strength of the nanocomposites was investigated by shear-lap tests (in-vitro longitudinal shear) on two PMMA sheets as bone substitute, bonded together because of the difficulty in obtaining bone samples of the appropriate geometry and size (Figure 9(c)). The concentration of curing agents was optimized by carrying out shear lap tests on PDA polymer with two curing agents (FeCl₃ and KMnO₄) in low/medium/high concentrations (2 mmol/L, 20 mmol/L and 200 mmol/L) (Figure 9(a)). After the best concentration for each curing agent was decided upon (20 mmol/L), the curing condition was optimized by carrying out shear lap tests on the PDA polymer with the two optimized curing agents and also ethanol as additive (Figure 9(b)). Lastly, the optimized PDA adhesive (20 mmol/L FeCl₃/H₂O+10µl ethanol) and PDA nanocomposite adhesive (PDA adhesive+10wt% HA) were analyzed on PMMA sheets in comparison to KRYPTONITE™ (Figure 9(d)). From our investigation, at the early stage (≤1 day) KRYPTONITE™ samples showed almost full strength, yielding a mean lap shear strength of 614 KPa (SD = 50 KPa, n = 5) and 787 KPa (SD = 96 KPa, n = 5) after 4 hours and 1day curing time respectively (Figure 9(d)). Such a high lap shear strength would require the surgeon to use an electrical saw if emergency re-entry was required, putting the patient at elevated risk. However, PDA adhesive has a controllable curing speed, allowing setting to take place over one week due to its unique structures and functionalities. When curing with FeCl₃, a much lower relative shear strength (509 KPa, SD = 37 KPa, n = 5) can be achieved within one day, for emergency convenience, but eventually forming a high shear strength of 743 KPa (SD = 39 KPa, n = 5) after 7 days curing which is substantial for sternal wound closure. The PDA adhesive with 10% HA reinforcement showed significant improvement to around 850 KPa (SD = 70 KPa, n = 5) after 7 days curing (Figure 9(d)). The increment in the adhesive strength is due to the strong hydrogen bonding interactions of large numbers of end functionality (catechol groups) of the hyperbranched polymer along with polar groups in the HA systems with the hydroxyl groups on the adherent substrate (Figure 9).

Ex-vivo compression (Figure 10(a) and (b)) and lateral distraction measurements (Figure 10(c) and (b)) yielded the mechanical strength by loading compression and lateral distraction on PDA nanocomposite adhesive bonding the two lamb sternal halves. After introducing HA particles into the hydrophobic PDA polymer, the adhesive is less fluid, making it better for sticking to the bone and not prematurely slipping away or penetrating into the bone pores. KRYPTONITE™ yielded a mean compression strength of 514 KPa (SD = 97 KPa, n = 5) which is nearly the full strength because of the fast curing rate (Figure 11(a)) and a mean lateral distraction strength of 255 KPa (SD = 57 KPa, n = 5) (Figure 11(b)) in 1 day curing time. However, PDA adhesive has a controllable curing speed within one week and a relatively lower adhesion strength in one day when loading compression (251 KPa, SD = 52 KPa, n = 5) (Figure 11(a)) and lateral distraction (171 KPa, SD = 45 KPa, n = 5) (Figure 11(b)) force but eventually increased to a high compression strength of 623 KPa (SD = 71 KPa, n = 5) (Figure 6(a)) and lateral distraction strength of 633 KPa (SD = 52 KPa, n = 5) (Figure 11(b)) after 7 days curing time which is substantial to withstand the stresses in-vivo. Upon completion of the test, all samples showed an adhesive failure with the bone surfaces, rather than cohesive failure. *Quantifying Degradation (Weight loss)*

Weigh samples before placing in PBS. Heat bottles of PBS in oven, for 15-20 minutes, to 37°C. Place samples in PBS and replace in oven for time point to allow for degradation. Remove from PBS and test mechanical strength of samples. Compare to non-degraded samples for control. Weight loss along time. PDA shows appropriate degradable performance and degrade to almost 60% in one month time. Kryptonite almost do not degrade in one month time (Figure 12).

### Quantifying Cytoxicity

3T3 fibroblasts were cultured in DMEM supplemented with 10% FBS and 1% Pen/Step under standard sterile cell culture conditions at 37°C and 5% CO₂. The cells were seeded in 48 well-plates at a concentration of 10, 000 cells per well. 1 mg of the adhesives, with various crosslinkers, were added to the cells one day after seeding to result in a final concentration of 2 mg/ml. To assess the cell viability one, four and seven days post the addition of the adhesive, the AlamarBlue^{®} cell metabolic activity method was used as described previously. Cell viability was obtained by normalising metabolic activity to cells receiving no treatment. The PDA adhesive without a crosslinker and TISSEEL^{®} fibrin glue were used for comparisons. (Figure 13).

Hyperbranched polymers belong to a class of synthetic tree-like macromolecules called dendritic polymers. They are polymers with densely branched structure and a large number of end groups. Dendritic polymers include dendrimers which have completely branched star-like topologies and hyperbranched polymers which have imperfectly branched or irregular structures. Both dendrimer and hyperbranched polymer molecules are composed of repeating units emanating from a central core. The core is characterised by its functionality, which is the number of chemical bonds through which it can be connected to the external parts of the molecule. The functionality of the core is normally three (e.g. amine) or four (e.g. ethylenediamine). Through the bonds of the core, the layers of linear units (single monomers or linear chains) are attached to the core and each of these arms is terminated with the multifunctional branched unit. Larger molecules are created by adding shells of linear units to the end groups of the layer beneath. If all of these units are attached to the molecule perfectly, a dendrimer is formed. In contrast, the absence of any of these units in the molecule will result in a hyperbranched polymer structure.

Furthermore, the invention provides a synthesis platform suitable for the production of specifically designed adhesives that are demonstrated to deliver improved adhesive properties within the context of the invention. The methods provided herein are capable of synthesizing polymers with impressive biodegradable properties [intracellular] and the specific addition of modifiable functional groups, making attachment of moieties of a variety of other applications attachment a genuine prospect.

In particular, the Inventors have demonstrated the synthesis and characterisation of hyperbranched or linearly branched cationic polymers suitable for use as medical adhesives, and/or bone cements.

Polymer Synthesis & Characterisation -Copolycondensation reactions are not suitable for forming hyperbranced copolymers as vinyl monomers cannot be polymerized by these methods. However, SCVP can be used to produce such hyperbranched polymers. Typically, a vinyl monomer of structure AB can be used, where A is a vinyl group and B is a functional group that can be converted into an initiating group B* by an external stimulus. This species is known as an inimer. The activated species can be a "living" free radical, an electrophilic cationic moiety or a carbanion. Inimers often comprise halogen groups, whereby activation occurs by removing the halogen to form either a cation or a radical. Controlled/living radical polymerisation (CRP) is a widely used technique that allows the synthesis of defined polymer architectures through precise control of molecular weights and distributions. The architectures of polymers prepared by the CRP techniques are reported to be the linear, crosslinked, branched/dendritic structures. By precisely controlling the competition between chain growth and reversible chain termination, the polymers can grow slowly and effectively while branching is introduced by the multi-functional vinyl monomers in a controlled fashion and the cross linking reaction is delayed. DE-ATRP allows the simple use of readily available and inexpensive multi-functional vinyl momomers to synthesis soluble hyperbranced polymers with a high degree of branching and controlled chain structure via homo- and copolymerisations. The key to the strategy is to control competition between chain growth and revesible chain termination via a deactivation enhanced method. Thus the polymer chains can grow effectively and brancing is introduced by multifunctional vinyl monomer in a controlled fashion, leading to the formation of hyperbranced polymers rather than cross linked gel.

The adhesives of the invention are synthetic polymers that are synthesised by the process of Atom Transfer Radical Polymerisation (ATRP) or Reversible Addition Fragmentation Chain-transfer (RAFT) polymerization to yield hyperbranched or linearly branched polymers. Other synthetic possibilities include Michael Addition and click chemistry processes.

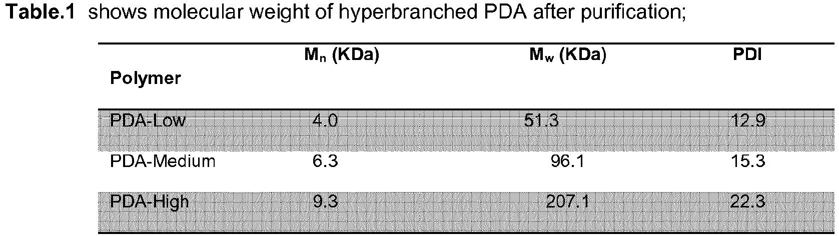

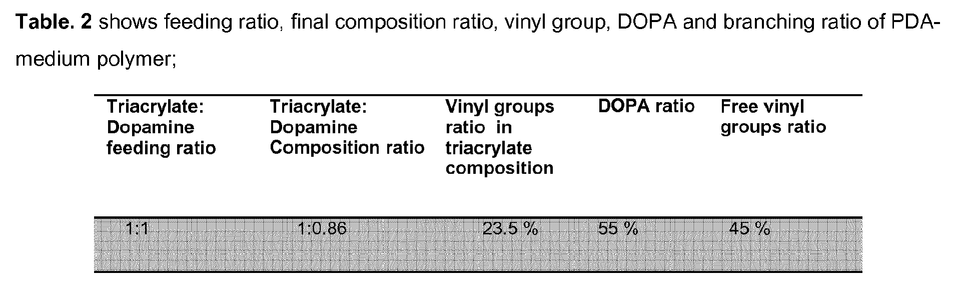

## Claims

1. A dendritic or hyperbranched polymer comprising a plurality of branches, wherein at least one of the branches comprises at least one catechol moiety that is oxidisible to a semiquinone moiety and/or a quinone moiety **characterised in that** the polymer further comprises at least one other functional group selected from the group consisting of: a vinyl functionality which may be an unsubstituted vinyl or a functionalized vinyl group, an amine functionality, a disulfide functionality, at least one ionisable amine, diamine, tri-amine, amino alcohol functionality, and/or combinations thereof and wherein optionally at least one of the plurality of polymeric branches terminates with a hydroxyl functionality or terminates with a halide selected from the group consisting of: F, CI, Br and I.

2. A dendritic or hyperbranched polymer according to claim 1 wherein the catechol moiety is derived from DOPA or dopamine.

3. A dendritic or hyperbranched polymer according to any preceding claim in which the dendritic or hyperbranched polymer is polydisperse and optionally is single cyclized or multi-cyclized.

4. A dendritic or hyperbranched polymer according to any preceding claim wherein the vinyl group or vinyl functionality is a methacrylate group which can be unsubstituted or substituted with a linear or branched Ci_6 alkyl.

5. A dendritic or hyperbranched polymer according to claim 1 wherein the polymer is derived from co-polymerisation of dopamine HCI monomer and trimethylol propane triacrylate monomer (TMPTA) and has the ¹H NMR spectrum shown in Figure 7.

6. A dendritic or hyperbranched polymer according to claim 1 wherein the polymer is poly-dopamine-co-acrylate.

7. A dendritic or hyperbranched polymer according to any preceding claim, wherein at least one of the plurality of branches are crosslinked with a crosslinking agent or crosslinker system, which can optionally be used with a crosslinking promoter.

8. A dendritic or hyperbranched polymer according to claim 7 wherein the branches are crosslinked with fibrinogen, chitosan or a crosslinker system that is promoted by an oxidation reaction.

9. A dendritic or hyperbranched polymer according to claim 8 wherein the oxidation promoted crosslinker system is permanganate, Fe(III), HPR (Horseradish peroxidase) and peroxide, or combinations thereof.

10. Polymer according to any preceding claim or a composition of a polymer according to any preceding claim, for use as medical adhesive or bone cement.

11. Polymer or composition for use according to claim 10 wherein the bone cement is a cement for sternum bone closure or other orthopaedic operating procedure.

12. Use of a polymer according to any one of claims 1 to 1 1 in the manufacture of a medical adhesive.

13. A process for synthesising a dendritic or hyperbranched polymer comprising at least one catechol moiety by polymerising a divinyl monomer or a triacrylate with other optional polymerisable monomers and/or initiators including those having at least one catechol moiety under suitable polymerisation conditions to effect polymerisation of the monomer by a Michael Addition, a click chemistry or a living polymerisation process.

14. A method of curing an adhesive or bone cement comprising a polymer as defined in any one of claims 1 to 9 comprising the step of crosslinking the plurality of polymer branches with a crosslinker system as defined in claim 7.

## Patentansprüche

1. Ein dendritisches oder hyperverzweigtes Polymer, umfassend mehrere Zweige, wobei mindestens einer der Zweige mindestens eine Catecholeinheit umfasst, die zu einer Semichinoneinheit und/oder einer Chinoneinheit oxidierbar ist, **dadurch gekennzeichnet, dass** das Polymer ferner mindestens eine andere funktionelle Gruppe umfasst, ausgewählt aus der Gruppe bestehend aus: einer Vinylfunktionalität, die ein unsubstituiertes Vinyl oder eine funktionalisierte Vinylgruppe sein kann, einer Aminfunktionalität, einer Disulfidfunktionalität, mindestens einem ionisierbaren Amin, Diamin, tri-Amin, einer Aminoalkoholfunktionalität und/oder Kombinationen davon, und wobei gegebenenfalls mindestens einer der mehreren polymeren Zweige mit einer Hydroxylfunktionalität endet oder mit einem Halogenid ausgewählt aus der Gruppe bestehend aus: F, Cl, Br und I endet.

2. Ein dendritisches oder hyperverzweigtes Polymer gemäß Anspruch 1, wobei die Catecholeinheit von DOPA oder Dopamin abgeleitet ist.

3. Ein dendritisches oder hyperverzweigtes Polymer gemäß einem der vorangehenden Ansprüche, wobei das dendritische oder hyperverzweigte Polymer polydispers ist und gegebenenfalls einfach cyclisiert oder mehrfach cyclisiert ist.

4. Ein dendritisches oder hyperverzweigtes Polymer gemäß einem der vorangehenden Ansprüche, wobei die Vinylgruppe oder Vinylfunktionalität eine Methacrylatgruppe ist, die unsubstituiert oder mit einem linearen oder verzweigten C₁₋₆Alkyl substituiert sein kann.

5. Ein dendritisches oder hyperverzweigtes Polymer gemäß Anspruch 1, wobei das Polymer aus der Co-Polymerisation von Dopamin HCl-Monomer und Trimethylolpropantriacrylat-Monomer (TMPTA) abgeleitet ist und das ¹H NMR Spektrum wie in Figur 7 gezeigt aufweist.

6. Ein dendritisches oder hyperverzweigtes Polymer gemäß Anspruch 1, wobei das Polymer Poly-dopamin-co-acrylat ist.

7. Ein dendritisches oder hyperverzweigtes Polymer gemäß einem der vorangehenden Ansprüche, wobei mindestens einer der mehreren Zweige mit einem Vernetzungsmittel oder Vernetzersystem, die gegebenenfalls mit einem Vernetzungsbeschleuniger verwendet werden können, vernetzt ist.

8. Ein dendritisches oder hyperverzweigtes Polymer gemäß Anspruch 7, wobei die Zweige mit einem Fibrinogen, Chitosan oder einem Vernetzersystem vernetzt sind, das durch eine Oxidationsreaktion beschleunigt wird.

9. Ein dendritisches oder hyperverzweigtes Polymer gemäß Anspruch 8, wobei das Vernetzersystem, das durch Oxidation beschleunigt wird, Permanganat, Fe(III), HPR (Meerrettichperoxidase) und Peroxid, oder Kombinationen davon ist.

10. Polymer gemäß einem der vorangehenden Ansprüche oder eine Zusammensetzung eines Polymers gemäß einem der vorangehenden Ansprüche zur Verwendung als medizinischer Klebstoff oder Knochenzement.

11. Polymer oder Zusammensetzung zur Verwendung gemäß Anspruch 10, wobei der Knochenzement ein Zement für Brustbeinverschluss oder ein anderes orthopädisches Operationsverfahren ist.

12. Verwendung eines Polymers gemäß einem der Ansprüche 1 bis 11 bei der Herstellung eines medizinischen Klebstoffs.

13. Ein Verfahren zur Synthetisierung eines dendritischen oder hyperverzweigten Polymers, das mindestens eine Catecholeinheit umfasst, durch Polymerisieren eines Divinylmonomers oder eines Triacrylats gegebenenfalls mit anderen polymerisierbaren Monomeren und/oder Initiatoren, die solche mit mindestens einer Catecholeinheit beinhalten, unter geeigneten Polymerisationsbedingungen, um Polymerisation des Monomers durch eine Michael-Addition, eine Click-Chemie oder ein Lebend-Polymerisationsverfahren durchzuführen.

14. Ein Verfahren zum Härten eines Klebstoffs oder Knochenzements, der ein Polymer umfasst, wie in einem der Ansprüche 1 bis 9 definiert, umfassend den Schritt des Vernetzens der mehreren Polymerzweige mit einem Vernetzungssystem wie in Anspruch 7 definiert.

## Revendications

1. Polymère dendritique ou hyper-ramifié comprenant une pluralité de branches, dans lequel au moins l'une des branches comprend au moins une fraction catéchol qui est oxydable en une fraction semi-quinone et/ou une fraction quinone, **caractérisé en ce que** le polymère comprend en outre au moins un autre groupe fonctionnel sélectionné dans le groupe constitué par une fonctionnalité vinyle qui peut être un vinyle non substitué ou un groupe vinyle fonctionnalisé, une fonctionnalité amine, une fonctionnalité disulfure, au moins l'une d'une amine, diamine, tri-amine ionisable, une fonctionnalité alcool aminé, et/ou leurs combinaisons, et dans lequel optionnellement au moins l'une de la pluralité de branches du polymère se termine par une fonctionnalité hydroxyle ou se termine par un halogénure sélectionné dans le groupe constitué de F, Cl, Br et I.

2. Polymère dendritique ou hyper-ramifié selon la revendication 1, dans lequel la fraction catéchol est dérivée de DOPA ou de dopamine.

3. Polymère dendritique ou hyper-ramifié selon l'une quelconque des revendications précédentes, dans lequel le polymère dendritique ou hyper-ramifié est poly-dispersé et optionnellement est monocyclique ou multi-cyclique.

4. Polymère dendritique ou hyper-ramifié selon l'une quelconque des revendications précédentes, dans lequel le groupe vinyle ou la fonctionnalité vinyle est un groupe méthacrylate qui peut être non substitué ou substitué avec un alkyle C₁₋₆ linéaire ou ramifié.

5. Polymère dendritique ou hyper-ramifié selon la revendication 1, dans lequel le polymère est dérivé de la copolymérisation de monomère dopamine HCl et de monomère triacrylate de triméthylolpropane (TMPTA) et a un spectre ¹H NMR représenté en figure 7.

6. Polymère dendritique ou hyper-ramifié selon la revendication 1, dans lequel le polymère est un polydopamine-co-acrylate.

7. Polymère dendritique ou hyper-ramifié selon l'une quelconque des revendications précédentes, dans lequel au moins l'une de la pluralité de branches est réticulée à l'aide d'un agent de réticulation ou d'un système de réticulation qui peut optionnellement être utilisé avec un promoteur de réticulation.

8. Polymère dendritique ou hyper-ramifié selon la revendication 7, dans lequel les branches sont réticulées avec du fibrinogène, du chitosane ou un système de réticulation qui est favorisé par une réaction d'oxydation.

9. Polymère dendritique ou hyper-ramifié selon la revendication 8, dans lequel le système de réticulation favorisé par une oxydation est du permanganate, du Fe(III), du HPR (peroxydase de raifort) et du peroxyde, ou une de leurs combinaisons.

10. Polymère selon l'une quelconque des revendications précédentes ou composition de polymère selon l'une quelconque des revendications précédentes, pour utilisation comme adhésif médical ou comme ciment osseux.

11. Polymère ou composition pour utilisation selon la revendication 10, dans lequel le ciment osseux est un ciment pour la fermeture du sternum ou une autre procédure d'opération orthopédique.

12. Utilisation d'un polymère selon l'une quelconque des revendications 1 à 11 dans la fabrication d'un adhésif médical.

13. Procédé de synthèse d'un polymère dendritique ou hyper-ramifié comprenant au moins une fraction catéchol, en polymérisant un monomère di-vinylique ou un triacrylate avec d'autres monomères polymérisables optionnels et/ou des initiateurs comprenant ceux comportant au moins une fraction catéchol dans des conditions de polymérisation appropriées pour effectuer une polymérisation du monomère par une addition de Michael, une chimie clic ou un procédé de polymérisation vivante.

14. Procédé pour durcir un adhésif ou un ciment osseux comprenant un polymère tel que défini dans l'une quelconque des revendications 1 à 9, comprenant l'étape consistant à réticuler la pluralité de branches du polymère à l'aide d'un système de réticulation tel que défini dans la revendication 7.
